# EUROPEAN PATENT SPECIFICATION

(11) **EP 3 641 710 B1**
(45) Date of publication and mention of the grant of the patent: **27.10.2021**
(21) Application number: 18739644.5
(22) Date of filing: 12.06.2018
(51) Int. Cl.: A61F 13/02

(54) **NEGATIVE PRESSURE WOUND THERAPY ARTICLE WITH FEATURES**
UNTERDRUCKWUNDTHERAPIEVORRICHTUNGSARTIKEL MIT MERKMALEN
ARTICLE DE THÉRAPIE DES PLAIES PAR PRESSION NÉGATIVE DOTÉ D'ÉLÉMENTS

(30) Priority: 22.06.2017 US 201762523439 P; 14.12.2017 US 201762598686 P
(43) Date of publication of application: 29.04.2020
(73) Proprietor: 3M Innovative Properties Company, St. Paul, MN 55133-3427 (US)
(72) Inventor: MCNULTY, Amy K., Saint Paul Minnesota 55133-3427 (US); FITZSIMONS, Robert T. Jr., Saint Paul Minnesota 55133-3427 (US); HALVERSON, Kurt J., Saint Paul Minnesota 55133-3427 (US); BAKER, Bryan A., Saint Paul Minnesota 55133-3427 (US); NINKOVIC, Jana, Saint Paul Minnesota 55133-3427 (US); LIU, Jie, Saint Paul Minnesota 55133-3427 (US); DAI, Minghua, Saint Paul Minnesota 55133-3427 (US); ZHANG, Wei, Saint Paul Minnesota 55133-3427 (US); CLARKE, Graham M., Saint Paul Minnesota 55133-3427 (US); SGOLASTRA, Federica, Saint Paul Minnesota 55133-3427 (US)
(74) Representative: Mathys & Squire
(86) International application number: PCT/IB2018/054276
(87) International publication number: WO 2018/234931

(56) References cited:
- WO-A1-2014/028470
- WO-A1-2015/179235
- WO-A2-2013/032683

## Description

### BACKGROUND

Clinical studies and practice have shown that providing a negative pressure in proximity to a tissue site promotes the growth of new tissues at the tissue site. The application of negative pressure is successful in treating wounds. This treatment (frequently referred to in the medical community as "negative pressure wound therapy (NPWT)," "reduced pressure therapy," or "vacuum therapy") provides a number of benefits, including faster healing and increased formulation of granulation tissue. Typically, reduced pressure is applied to tissues through a foam, a pad or other manifolding device, such as gauze. The manifolding device typically contains cells, pores or other openings that are capable of distributing reduced pressure to the tissue and channeling fluids that are drawn from the tissue. The porous pad often is incorporated into a dressing having other components that facilitate treatment.

Other known NPWT articles are discussed in U.S. Patent Nos. 7,494,482; 8,057,447; 8,889,243 and 9,107,989.

### SUMMARY

In one aspect, the present disclosure provides an article, comprising: a network of interconnected polymeric strands; wherein each of the interconnected polymeric strands has a first surface adapted to contact a tissue site; wherein at least one of the interconnected polymeric strand has a plurality of protrusions extending from the first surface of the interconnected polymeric strands; wherein only one of any two adjacent polymeric strands has a plurality of protrusions extending from the first surface of the interconnected polymeric strands; and wherein at least one of the interconnected polymeric strands is non-linear; and a plurality of openings between adjacent interconnected polymeric strands.

In another aspect, the present disclosure provides a system, comprising: the article of present application; and a reduced pressure source fluidly connected to the opening of the article to deliver the reduced pressure through the opening, between the protrusions, and to the tissue site.

Various aspects and advantages of exemplary embodiments of the present disclosure have been summarized. The above Summary is not intended to describe each illustrated embodiment or every implementation of the present disclosure. Further features and advantages are disclosed in the embodiments that follow. The Drawings and the Detailed Description that follow more particularly exemplify certain embodiments using the principles disclosed herein.

### BRIEF DESCRIPTION OF THE DRAWINGS

The disclosure may be more completely understood in consideration of the following detailed description of various embodiments of the disclosure in connection with the accompanying figures, in which:
FIG. 1 illustrates an article according to an embodiment of the present invention.
FIG. 2 illustrates a top view of an article according to an embodiment of the present invention.
FIG. 3 illustrates the shape of the protrusions of the article according to an embodiment of the present invention.
FIG. 4 illustrates a schematic cross-section side view of an article according to an embodiment of the present invention.
FIG. 5 illustrates a reduced pressure treatment system according to an embodiment of the present invention.
FIG. 6 is a scanning electron microscope image of the article of Example 1.
FIG. 7 is a scanning electron microscope image of the article of Example 3.

While the above-identified drawings, which may not be drawn to scale, set forth various embodiments of the present disclosure, other embodiments are also contemplated, as noted in the Detailed Description. In all cases, this disclosure describes the claimed invention by way of representation of exemplary embodiments and not by express limitations. It should be understood that numerous other modifications and embodiments can be devised by those skilled in the art, which fall within the scope of this invention.

### DETAILED DESCRIPTION

Before any embodiments of the present disclosure are explained in detail, it is understood that the invention is not limited in its application to the details of use, construction, and the arrangement of components set forth in the following description. The invention is capable of other embodiments and of being practiced or of being carried out in various ways that will become apparent to a person of ordinary skill in the art upon reading the present disclosure. Also, it is understood that the phraseology and terminology used herein is for the purpose of description and should not be regarded as limiting. The use of "including," "comprising," or "having" and variations thereof herein is meant to encompass the items listed thereafter and equivalents thereof as well as additional items. It is understood that other embodiments may be utilized and structural or logical changes may be made without departing from the scope of the present disclosure.

As used in this Specification, the recitation of numerical ranges by endpoints includes all numbers subsumed within that range (e.g. 1 to 5 includes 1, 1.5, 2, 2.75, 3, 3.8, 4, and 5, and the like).

Unless otherwise indicated, all numbers expressing quantities or ingredients, measurement of properties and so forth used in the Specification and embodiments are to be understood as being modified in all instances by the term "about." Accordingly, unless indicated to the contrary, the numerical parameters set forth in the foregoing specification and attached listing of embodiments can vary depending upon the desired properties sought to be obtained by those skilled in the art utilizing the teachings of the present disclosure. At the very least, and not as an attempt to limit the application of the doctrine of equivalents to the scope of the claimed embodiments, each numerical parameter should at least be construed in light of the number of reported significant digits and by applying ordinary rounding techniques.

The article of the present application is well suited to promote tissue growth at the tissue site yet prevent in-growth of new tissue into the article. The article of the present application can help to deliver a significant portion of microstrain to the wound site by the architecture of the article, for example, the surface morphology of the article and thus may allow for lower pressure settings for NPWT to be used (for example, -75 mmHg vs -125 mmHg). This may allow a longer battery life of the NPWT system and the use of smaller pumps for the NPWT.

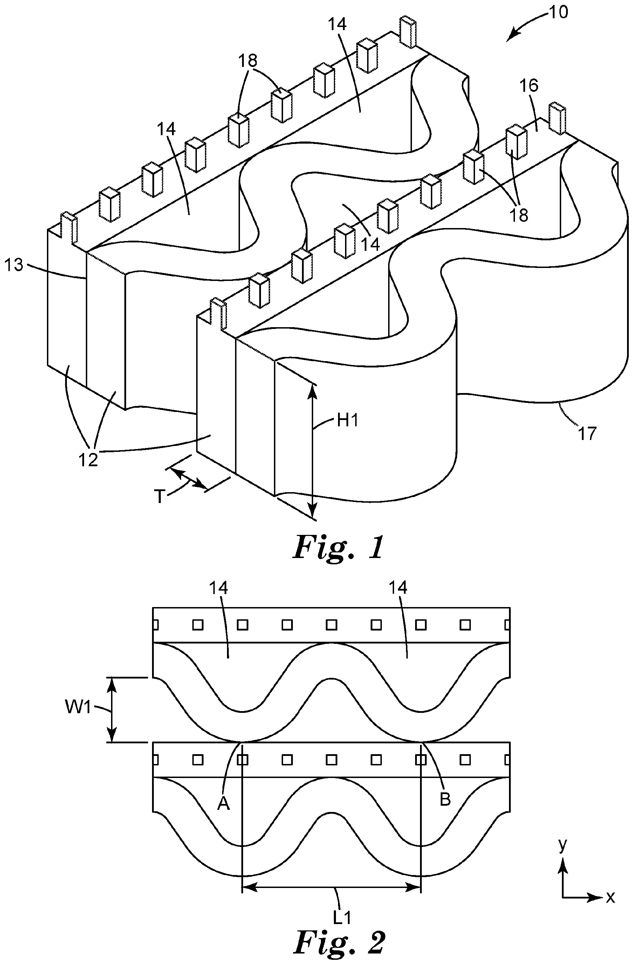

Referring to FIG. 1, an article 10 according to an embodiment of the present invention includes a network of interconnected polymeric strands, or sheets 12 and a plurality of openings 14 between adjacent polymeric strands. Polymeric strands 12 can be connected at connections 13. Typically, there are a plurality of connections 13 between adjacent strands. Polymeric strands 12 have a tissue contact surface 16 as a first surface and a second surface 17 opposite the first surface. At least one of the interconnected polymeric strand has a plurality of protrusions 18 extending from the first surface 16 of the interconnected polymeric strands 12. In the embodiment of FIG. 1, only one of any two adjacent polymeric strands has a plurality of protrusions extending from the first surface of the interconnected polymeric strands. In some embodiments, the protrusions 18 do not substantially contact each other (i.e., at least 50 (at least 55, 60, 65, 70, 75, 80, 85, 90, 95, 99, or even 100) percent by number do not contact each other). The openings 14 form or provide open fluid channels from the first surface 16 of the network of polymeric strands to a second surface opposite first surface 16. Through the open fluid channels, openings 14 are typically used to allow reduced pressure to be applied to a tissue site.

Referring more specifically to FIG. 1, the height, H1, of each polymeric strand 12 may be up to 2000 micrometers, up to 1500 micrometers, up to 1000 micrometers, up to 500 micrometers, or up to 400 micrometers. In some embodiments, the height, H1, of each polymeric strand 12 may be no less than 100 micrometers, no less than 200 micrometers, or no less than 300 micrometers. In some embodiments, the height, H1, of each polymeric strand 12 may be between 100 and 2000 micrometers, between 200 and 1500 micrometers, between 300 and 1000 micrometers, between 300 and 500 micrometers or between 300 and 400 micrometers. In some embodiments, the height, H1, of each polymeric strand 12 may be between 100 and 750 micrometers or between 100 and 5500 micrometers. In some embodiments, the width, T, of each polymeric strand 12 may have an average width up to 800 micrometers, up to 500 micrometers, up to 400 micrometers, or up to 250 micrometers. In some embodiments, the thickness, T, of each polymeric strand 12 may have an average width no less than 10 micrometers. In some embodiments, the thickness, T, of each polymeric strand 12 may have an average width in a range from 10 micrometers to 800 micrometers, from 10 micrometers to 500 micrometers, from 10 micrometers to 400 micrometers, or 10 micrometers to 250 micrometers, from 100 micrometers to 750 micrometers, from 200 micrometers to 750 micrometers.

In some embodiments, the article comprising interconnected polymeric strands has an average thickness not greater than 5 mm. In one embodiment of the present invention, the height and thickness of the interconnected polymeric strands 12 is uniform for a particular article 10. In other embodiments, the height and thickness of the interconnected polymeric strands 12 may be different. For example, the interconnected polymeric strands 12 having different height. Similarly, thickness of the interconnected polymeric strands 12 may vary. In some, embodiments, the interconnected polymeric strands 12 may have a range of thicknesses, for example, the interconnected polymeric strands 12 tends to be thinnest where it abuts an opening.

In some embodiments, the article comprising interconnected polymeric strands has a thickness up to 2 mm, up to 1 mm, up to 500 micrometers, up to 250 micrometers, up to 100 micrometers, up to 75 micrometers, up to 50 micrometers, or up to 25 micrometers. In some embodiments, the article comprising interconnected polymeric strands has a thickness no less than 10 micrometers. In some embodiments, the article comprising interconnected polymeric strands has a thickness in a range from 10 micrometers to 2 mm, 10 micrometers to 1 mm, 10 micrometers to 750 micrometers, 10 micrometers to 500 micrometers, 10 micrometers to 250 micrometers, 10 micrometers to 100 micrometers, 10 micrometers to 75 micrometers, 10 micrometers to 50 micrometers, or 10 micrometers to 25 micrometers. In some embodiments, the article comprising interconnected polymeric strands has an average thickness in a range from 250 micrometers to 5 mm.

In some embodiments, at least one of the interconnected polymeric strands 12 may be non-linear. In some embodiments, at least 25% of the interconnected polymeric strands 12 may be non-linear. In some embodiments, at least 50% of the interconnected polymeric strands 12 may be non-linear. In some embodiments, at least 75% of the interconnected polymeric strands 12 may be non-linear. In some embodiments, essentially all the interconnected polymeric strands 12 may be non-linear. In some embodiments, all of the interconnected polymeric strands 12 may be non-linear. In some embodiments, the non-linear polymeric strand may have a shape of a curve. In some embodiments, the non-linear polymeric strand may have a shape of a sinusoidal curve. In other embodiments, at least one of the interconnected polymeric strands 12 may be linear. In some other embodiments, 25% to 75% of the interconnected polymeric strands 12 may be linear. In some other embodiments, 50% to 75% of the interconnected polymeric strands 12 may be linear. In certain embodiments, the network of interconnected polymeric strands may include alternating non-linear polymeric strands and linear polymeric strands, as shown in FIG. 2. In some embodiments, the interconnected polymeric strands 12 are oriented in the same direction, for example, x direction as illustrated in FIG. 2. In some embodiments, the interconnected polymeric strands 12 do not substantially cross over each other (i.e., at least 50 (at least 55, 60, 65, 70, 75, 80, 85, 90, 95, 99, or even 100) percent by number do not cross over each other).

In some embodiments, aspect ratio (a ratio of the length to the width) of the openings 14 may be greater than 1:1, 1.5:1, 2:1, 3:1 or 5:1. In some embodiments, aspect ratio (a ratio of the length to the width) of the openings 14 may be in a range from 1:1 to 100:1, 1:1 to 75:1, 1:1 to 50:1, 1:1 to 25:1, 2:1 to 100:1 2:1 to 75:1, 2:1 to 50:1, 2:1 to 25:1, or 2:1 to 10:1. The length, L1, of an opening 14 illustrated in FIG. 2 is the longest lateral distance parallel to x direction, for example, the length between connections A and B. If the non-linear polymeric strand has a shape of a sinusoidal curve, the length of the opening 14 equals the wavelength of the sinusoidal curve. The width, W1, of an opening 14 illustrated in FIG. 2 is the longest distance parallel to y direction. If the non-linear polymeric strand has a shape of a sinusoidal curve, the width of the opening 14 can be up to two times amplification of the sinusoidal curve. Openings 14 of the article may have a range of L1 and W1 values as a result in part of variable spacing of the connections A and B. In some embodiments, the openings have widths, W1, up to 10 mm, up to 1 mm or up to 0.5 mm. In some embodiments, the openings have widths, W1, at least 5 micrometers or at least 10 micrometers. In some embodiments, the openings have widths, W1, in a range from 5 micrometers to 1 mm or from 10 micrometers to 0.5 mm or from 50 micrometers to 700 micrometers. In some embodiments, the openings have lengths, L1, up to 10 mm or up to 1 mm. In some embodiments, the openings have lengths, L1, at least 100 micrometers. In some embodiments, the openings have lengths, L1, in a range from 100 micrometers to 10 mm or from 100 micrometers to 1 mm. In some embodiments, the openings have lengths, L1, in a range from 400 micrometers to 6 mm or from 800 micrometers to 6 mm. FIGs 1 and 2 are idealized illustrations of one embodiment of the present application. In some embodiments, the openings 14 may have irregularly formed perimeters. This can mean that the openings have irregular shapes (that is, no lines of symmetry). They may have edges that are not smooth (e.g., jagged or feathery edges). Irregularly formed openings can also have a variety of thicknesses of the polymeric strands surrounding the openings.

In some embodiments, openings 14 may have any suitable shape, for example, a shape selected from shapes of an ellipse, oval, pointed oval (or lens or vesical piscis shape), diamond, ½ ellipse, ½ oval, ½ lens, triangle, etc. In some embodiments, the openings of the mechanical fastening nets described herein have at least two pointed ends. In some embodiments, at least some of the openings are elongated with two pointed ends. In some embodiments, at least some of the openings are elongated with two opposed pointed ends. In some embodiments, at least some of the openings are ovals.

In some embodiments, the article described herein have a total open area for each of the first and second, generally opposed surfaces of not greater than 50 (in some embodiments, not greater than 45, 40, 35, 30, 25, 20. 15, 10, 5, 4 3, 2, 1, 0.75, 0.5, 0.25, or even not greater than 0.1) percent of the total area of the respective surface. In some embodiments, for at least a majority of the openings of the article described herein, the maximum area of each opening is not greater than is 5 (in some embodiments, not greater than 2.5, 2, 1, 0.5, 0.1, 0.05, 0.01, 0.075, or even not greater than 0.005)mm². Individual openings range from 0.005 mm² to 5 mm². In some embodiments, the article according to the present disclosure have in a range from 50,000 to 6,000,000 (in some embodiments, 100,000 to 6,000,000, 500,000 to, 6,000,000, or even 1,000,000 to 6,000,000) openings/m².

In some embodiments, the tensile strength of the article parallel to x direction is greater than the tensile strength of the article parallel to y direction. Therefore, the article is easier to be stretched in y direction than in x direction. In some embodiments, the tensile strength of the article parallel to x direction is at least 2.23 MPa, at least 2.25 MPa, at least 2.5 MPa or at least 3.0 MPa. In some embodiments, the tensile strength of the article parallel to x direction is up to 5.42 MPa, up to 5.3 MPa, up to 5.0 MPa, or up to 4.5 MPa. In some embodiments, the tensile strength of the article parallel to x direction is from 2.23 MPa to 5.42 MPa., from 2.5 MPa to 5.0 MPa or from 3.0 MPa to 4.5 MPa. The Young's modulus of the article is up to 10.6 MPa, up to 10.0 MPa, up to 9.0 MPa, or up to 8.0 MPa. The Young's modulus of the article is at least 3.85 MPa, at least 4.0 MPa has a range from 3.85 MPa to 10.6 MPa in a direction parallel to x direction. The x direction and y direction of an article are as shown in FIG. 2. The x direction of an article is the direction in which the polymeric strands are oriented and the y direction of an article is perpendicular to the x direction.

The shape, sizing, and spacing of the protrusions 18 may vary depending upon the particular tissue site being treated, the type of material from which the protrusions 18 and polymeric strands are made, and the amount of reduced pressure being applied to the tissue site. For example, for tissue sites that are highly exuding, it may be advantageous to position the protrusions farther apart or reduce the density of protrusions on the first surface to maintain adequate distribution channels between the protrusions 18. In one embodiment of the present invention, the shape, sizing and spacing of the protrusions 18 is uniform for a particular article 10. In other embodiments, the shape, sizing, and spacing of the protrusions 18 may be different. For example, protrusions 18 having different cross-sectional shapes may be disposed on the first surface. Similarly, the sizing and spacing of the protrusions 18 may vary to supply selected portions of the tissue site with different (more or less) reduced pressure and different flow rate for exudates withdrawn.

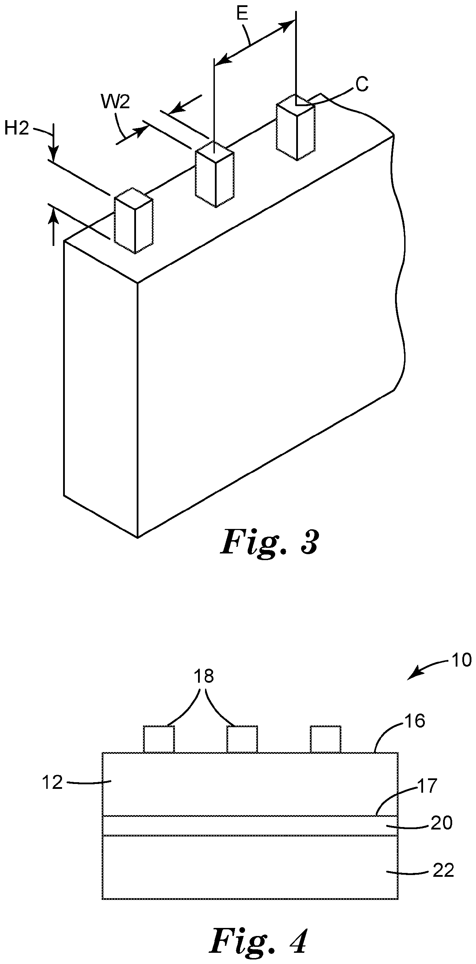

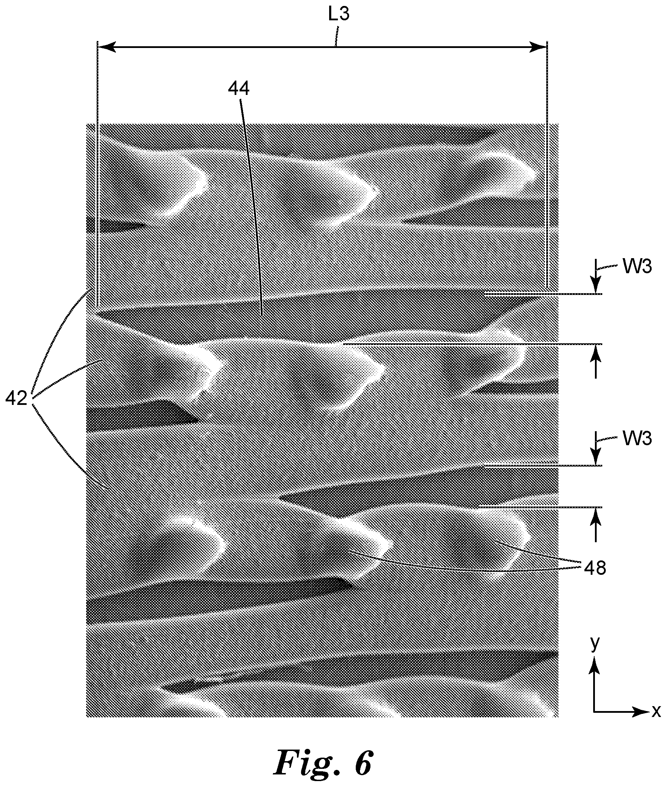

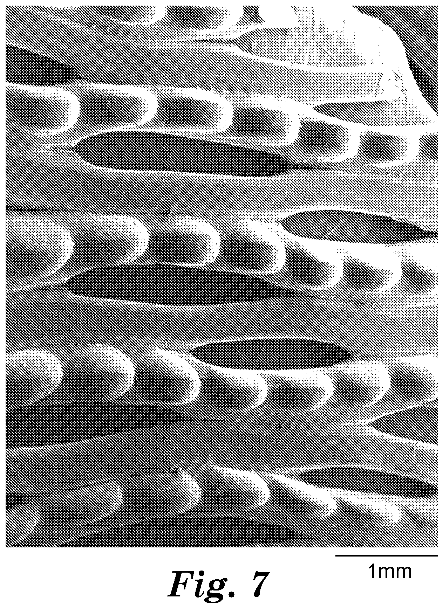

In some embodiments the tissue contacting surface (or tip) of a feature is flat (as shown in FIG. 3). When the tissue contacting surface (or tip) of a feature is flat, the edges of the surface can be sharp or rounded. In other embodiments the tissue contacting surface (or tip) of the feature is rounded to provide a dome shape (see FIG. 6 and FIG. 7).

Referring more specifically to FIG. 3, the height, H2, of the protrusions 18 may be up to 1000 micrometers, up to 500 micrometers or up to 450 micrometers. In some embodiments, the height, H2, of the protrusions 18 may be at least 100 micrometers or at least 200 micrometers. In some embodiments, the height, H2, of the protrusions 18 may be between 100 and 1000 micrometers, between 100 and 500 micrometers, between 200 and 500 micrometers or between 200 and 450 micrometers. The width, W2, of each feature may be up to 1000 micrometers, up to 900 micrometers, up to 800 micrometers, up to 700 micrometers or up to 600 micrometers. In some embodiments, the width, W2, of each feature may be at least 10 micrometers, at least 100 micrometers, at least 200 micrometers, at least 300 micrometers or at least 400 micrometers. In some embodiments, the width, W2, of each feature may be between 10 and 1000 micrometers, between 100 and 900 micrometers, between 200 and 800 micrometers, between 300 and 700 micrometers, or between 400 and 600 micrometers. In some embodiments, the width, W2, of each feature may be 500 micrometers. The width of the protrusions 18 illustrated in FIG. 3 is an edge length of the square since the cross-sectional shape of each protrusions 18 is square. If the protrusions 18 are circular in cross-sectional shape, the width of the protrusions 18 equals the diameter since the cross-sectional shape of each feature 18 is circular. For other cross-sectional shapes, the width is the average of the longest lateral distance through the centroid, C, of the cross section and the shortest lateral distance through the centroid of the cross section. It is generally preferred that the height of the protrusions 18 be no more than the width of the protrusions 18. More specifically, the ratio of height to width, H2:W2 of the protrusions 18, should be no more than 1:1. When the ratio of height to width, H2:W2 of the protrusions 18, is more than 1:1, the protrusions 18 is more prone to fall over the openings 14, thus reducing the fluid flow through the openings 14. The lateral, center-to-center spacing, E, between each feature 18 may be between 0.1 and 2.0 millimeters, between 0.5 and 1.5 millimeters or between 0.7 and 1.3 millimeters. The spacing of the protrusions 18 create distribution channels through which reduced pressure may be delivered to the tissue site and exudates withdrawn from the tissue site. The density of protrusions on the first surface may be less than 1,000/square inch to facilitate reduced pressure delivered to the tissue site and exudates withdrawn from the tissue site. In some embodiments, the density of protrusions on the first surface may be less than 1,000/square inch, less than 900/square inch, less than 800/square inch, less than 700/square inch, less than 600/square inch, or less than 500/square inch. In some embodiments, the number of protrusions in the article can be greater than the number of openings. For example, the ratio of the number of protrusions to the number of openings can be more than 1, 1.5, 2, 2.5, 3, 4, 5, or 10. In some embodiments, the article of the present disclosure can be a mechanical fastening net or a mechanical fastening sheet with protrusions.

In some embodiments, features 18 are oriented along the interconnected polymeric strands 12 as illustrated in FIG. 1. In other embodiments, protrusions 48 are oriented in substantially same orientation, for example x direction, as illustrated in FIG. 6.

The presence and sizing of the protrusions 18 allow the protrusions 18 to distribute reduced pressure to the tissue site, but prevent new tissue that grows at the tissue site from attaching to the protrusions 18 or growing into the spacing between protrusions 18. While new tissue growth may wrap around some of the protrusions 18, the new tissue is not capable of securing itself to the protrusions 18 since the base of each protrusions is anchored to the first surface 16.

In addition to distributing reduced pressure to the tissue site, the article 10 also serves to impart stresses and strains to the tissue site similar to those seen with cellular foam that traditionally has been used in reduced pressure systems. Other materials sometimes used in reduced pressure systems, such as gauze, do not have this effect on tissue. Unbound by the theory, the stresses and strains created by the article 10 are believed to cause micro-deformation of existing tissues and plays a significant role in the generation of new tissues at the tissue site. The amount of stress and strain imparted to a tissue site is determined by the amount of reduced pressure supplied to the tissue site and the surface morphology of the article that contacts the tissue site. As reduced pressure is applied, portions of the tissue site are pulled against the article 10, and more particularly against the protrusions 18, which results in the development of stresses and strains within the tissue. In some embodiments, the article of the present disclosure can be a mechanical fastening net or a mechanical fastening sheet with protrusions.

Referring to FIG. 4, in some embodiments, the article 10 may further include an adhesive layer 20 in contact with the second surface 17 of the interconnected polymeric strands 12. Suitable adhesive for use in the adhesive layer 20 of the article 10 can include any adhesive that provides acceptable adhesion to skin and is acceptable for use on skin (e.g., the adhesive should preferably be non-irritating and non-sensitizing). Suitable adhesives can be pressure sensitive and in certain embodiments have a relatively high moisture vapor transmission rate to allow for moisture evaporation. Suitable pressure sensitive adhesives include those based on acrylates, urethane, hyrdogels, hydrocolloids, block copolymers, silicones, rubber based adhesives (including natural rubber, polyisoprene, polyisobutylene, butyl rubber etc.) as well as combinations of these adhesives. The adhesive component may contain tackifiers, plasticizers, rheology modifiers as well as active components including for example an antimicrobial agent. Suitable adhesive can include those described in U.S. Patent Nos. 3,389,827; 4,112,213; 4,310,509; 4,323,557; 4,595,001; 4,737,410; 6,994,904 and International Publication Nos. WO 2010/056541; WO 2010/056543 and WO 2014/149718.

The article 10 may further include a cellular foam or another material 22 in contact with the adhesive layer, the adhesive layer in between the network of interconnected polymeric strands and the cellular foam or another material 22. In some other embodiments, the cellular foam or another material 22 that is positioned adjacent to or attached to the surface 17 of the interconnected polymeric strands opposite the protrusions 18. The use of a cellular foam or other material 22 increases the ability of the reduced pressure conduit 29 or the distribution adapter 35 to deliver and distribute reduced pressure to the article 10. The protrusions 18 and interconnected polymeric strands serve as a barrier to new tissue growth entering pores of the cellular foam or other material.

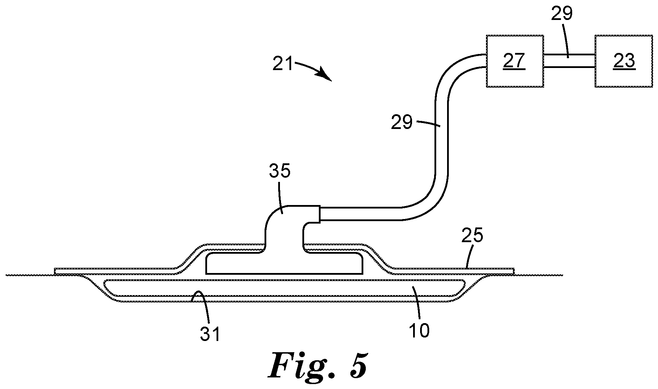

Referring to FIG. 5, a reduced pressure treatment system 21 according to an embodiment of the present invention includes a reduced pressure dressing, or article 10 fluidly connected to a reduced pressure conduit 29. The reduced pressure conduit 29 is fluidly connected to a reduced pressure source 23 such as a vacuum pump or another source of suction. The article 10 is placed against a tissue site 31 of a patient and is used to distribute a reduced pressure provided by the reduced pressure source 23. Typically, reduced pressure is maintained at the tissue site by placing an impermeable or semi-permeable cover 25 over the article 10 and the tissue site 31. The reduced pressure also serves to draw wound exudates and other fluids from the tissue site 31. A canister 27 may be fluidly connected to the reduced pressure conduit 29 and disposed between the article 10 and the reduced pressure source 23 to collect the fluids drawn from the tissue site 31. A distribution adapter 35 may be connected to the reduced pressure conduit 29 and positioned on the article 10 to aid in distributing the reduced pressure to the article 10.

In some embodiments, the interconnected polymeric strands 12 can include an elastomeric polymer. Elastomeric polymer can be any suitable elastomeric polymer, including but not limited to polyolefins and polyurethanes. In some embodiments, elastomeric polymer can be a medical grade material that is relatively impermeable to fluid flow. Alternatively, elastomeric polymer can be a semi-permeable material that allows select fluids or amounts of fluids to pass. In some embodiments, the interconnected polymeric strands 12 are formed from the same material as the protrusions 18. In some embodiments, the interconnected polymeric strands 12 can be formed from a different material as the protrusions 18. In some embodiments, the composition of interconnected polymeric strands 12 may be formed from different materials.

Some embodiments of the present wound-treatment methods can include positioning the article of present disclosure on a wound of a patient and applying a reduced pressure to the wound through the article (e.g., through the openings). Some embodiments of the present wound-treatment methods can further include: coupling a drape to skin adjacent the wound such that the drape covers the article and the wound, and forms a space between the drape and the wound. In some embodiments, positioning the article on the wound can include placing the article over the wound with the protrusions on the first surface facing the wound. In some embodiments, applying the reduced pressure to the wound comprises activating a vacuum source (e.g., reduced pressure source 23 of FIG. 5) that is coupled to the article. Some embodiments comprise: delivering a fluid to the wound through the article. In some embodiments, delivering a fluid comprises activating a fluid source that is coupled to the article.

### EXAMPLES

### Example 1

A net forming apparatus as generally depicted in United States Patent No. 8889243 (Hanschen, Fig. 1) was used with an extrusion die having offset orifices as generally described in United States Patent Application No. 2014/0234606 (Ausen, Fig. 5). The specific co-extrusion die of the example was assembled with a 20 shim repeating pattern of extrusion orifices. The thickness of the shims in the repeat sequence was 4 mils (0.102 mm) for shims with connection to the first cavity, the second cavity, and for the spacers which had no connection to either cavity. The shims were formed from stainless steel, with perforations cut by a wire electron discharge machining. The height of first and second extrusion orifices was cut to 30 mils (0.762 mm). The extrusion orifices with connection to the first cavity were aligned in a co-linear arrangement. The extrusion orifices with connection to the second cavity were aligned in a co-linear arrangement. The alignment of the first and second set of orifices was offset by 100%. Four spacer shims, followed by four shims with connection to the first cavity, followed by four spacer shims, followed by eight shims with connection to the second cavity comprised the shim stack sequence. The total width of the shim setup was 15 cm. The orifice width for first extrusion orifices leading to the first cavity was 0.408 mm, and the orifice width for second extrusion orifices leading to the second cavity was 0.816 mm. The land spacing between the first and second orifices was 0.408 mm.

The inlet fittings on the two end blocks were each connected to a conventional 1.25 inch (3.17 cm) single-screw extruder. The extruder feeding the first cavity was loaded with polyolefin elastomer resin pellets (obtained under the trade designation ENGAGE 8200 from the Dow Chemical Company, Midland, MI). The extruder feeding the second cavity was also loaded with polyolefin elastomer resin pellets (ENGAGE 8200). The extruder temperatures were set to 325 °F (162.8 °C) at the inlet and 375 °F (190.5 °C) at the outlet. The die temperature was set at 385 °F (196.1 °C). The extruder feeding the first cavity had a speed of 17.1 revolutions per minute (rpm) and the extruder feeding the second cavity had a speed of 9 rpm.

The net was extruded horizontally into a takeaway nip. The nip consisted of a metal nip roll surface and a silicone tool roll surface, with the temperatures set to 25 °C and 50 °C respectively. The cavities in the tool roll surface were circular in cross section with a diameter of 0.5 mm at the surface of the roll, a diameter of 0.3 mm at a depth of 1.5 mm, and a diameter of 0 mm at a depth of 2.0 mm. The cavities were arranged with center to center spacing of 1.35 mm in the axial roll direction and 1.35 mm spacing in the circumferential direction. The nip pressure was sufficient to fill the cavities to a height of 0.300 mm. Polymers were extruded from the two cavities at appropriate flow rates to make a net and the take away speed was 6 feet (1.83 m) per minute.

A scanning electron microscope image (SEM, 10kV x27, JCM-5000 NEOSCOPE Model from JEOL USA Inc., Peabody, MA) of the resulting net is shown in FIG. 6. The plurality of protrusions 48 were positioned on alternating strands 42 of the net and the interconnected strands were oriented in the x-direction. The image shows a plurality of openings 44 between adjacent polymeric strands 42. The tips of the protrusions were generally rounded. The net had a base thickness (strand height HI of Fig. 1) range of about 0.13-0.27 mm; strand width (T of Fig. 1) ranges of about 0.39-0.57 mm; openings with width (W3 of Fig. 5) ranges of about 0.15-0.43 mm and length (L3 of Fig. 5) ranges of about 2.90-3.39 mm; protrusions with width at base (W2 of Fig. 3) of about 0.33-0.54 mm and height (H2 of Fig. 3) ranges of about 0.20-0.37 mm; and feature spacing (E of Fig. 3) ranges of about 1.08-1.26 mm.

### Example 2

The net was prepared using the same general procedure as in Example 1. The specific co-extrusion die of the example was assembled with a 20 shim repeating pattern of extrusion orifices. The thickness of the shims in the repeat sequence was 4 mils (0.102 mm) for shims with connection to the first cavity, the second cavity, and for the spacers which had no connection to either cavity. The shims were formed from stainless steel, with perforations cut by a wire electron discharge machining. The height of first and second extrusion orifices was cut to 30 mils (0.762 mm). The extrusion orifices with connection to the first cavity were aligned in a co-linear arrangement. The extrusion orifices with connection to the second cavity were aligned in a co-linear arrangement. The alignment of the first and second set of orifices was offset by 100%. Four spacer shims, followed by four shims with connection to the first cavity, followed by four spacer shims, followed by eight shims with connection to the second cavity comprised the shim stack sequence. The total width of the shim setup was 15 cm. The orifice width for first extrusion orifices leading to the first cavity was 0.408 mm and the orifice width for second extrusion orifices leading to the second cavity was 0.816 mm. The land spacing between the first and second orifices was 0.408 mm.

The inlet fittings on the two end blocks were each connected to a conventional 1.25 inch (3.17 cm) single-screw extruder. The extruder feeding the first cavity was loaded with polyurethane thermoplastic resin pellets (obtained under the trade designation IROGRAN A 60 E 4902 from Huntsman Corporation, The Woodlands, TX). The extruder feeding the second cavity was also loaded with polyurethane thermoplastic resin pellets (IROGRAN A 60 E 4902). The extruder temperatures were set to 300 °F (148.9 °C) at the inlet and 330 °F (165.5 °C) at the outlet. The die temperature was set at 320 °F (160.0 °C). The extruder feeding the first cavity had a speed of 22.9 rpm and the extruder feeding the second cavity had a speed of 12 rpm.

The net was extruded vertically into a takeaway nip. The nip consisted of a metal nip roll surface and a silicone tool roll surface, with the temperatures set to 25 °C and 50 °C respectively. The cavities in the tool roll surface were circular in cross section with a diameter of 0.5 mm at the surface of the roll, a diameter of 0.3 mm at a depth of 1.5 mm, and a diameter of 0 mm at a depth of 2.0 mm. The cavities were arranged with center to center spacing of 1.35 mm in the axial roll direction and 1.35 mm spacing in the circumferential direction. The nip pressure was sufficient to fill the cavities to a height of 0.300 mm. Polymers were extruded from the two cavities at appropriate flow rates to make a net and the take away speed was 6 feet (1.83 m) per minute.

The resulting net had the plurality of protrusions positioned on alternating strands of the net and the interconnected strands were oriented in the x-direction. The net had a base thickness (strand height HI of Fig. 1) range of about 0.38-0.47 mm; strand width (T of Fig.1) ranges of about 0.32-0.46 mm; openings with width ranges of about 0.26-0.47 mm and length ranges of about 1.37-2.05 mm; protrusions with width at base (W2 of Fig. 3) of about 0.60-0.80 mm and height (H2 of Fig. 3) ranges of about 0.18-0.32 mm; and feature spacing (E of Fig. 3) ranges of about 1.24-1.57 mm.

### Example 3

A net forming apparatus as generally depicted in United States Patent No. 8889243 (Hanschen, Fig. 1) was used with an extrusion die having offset orifices as generally described in United States Patent Application No. 2014/0234606 (Ausen, Fig. 5). The specific co-extrusion die of the example was assembled with a 20 shim repeating pattern of extrusion orifices. The thickness of the shims in the repeat sequence was 4 mils (0.102 mm) for shims with connection to the first cavity, the second cavity, and for the spacers which had no connection to either cavity. The shims were formed from stainless steel, with perforations cut by a wire electron discharge machining. The height of first and second extrusion orifices was cut to 30 mils (0.762 mm). The extrusion orifices with connection to the first cavity were aligned in a co-linear arrangement. The extrusion orifices with connection to the second cavity were aligned in a co-linear arrangement. The alignment of the first and second set of orifices was offset by 100%. Four spacer shims, followed by four shims with connection to the first cavity, followed by four spacer shims, followed by eight shims with connection to the second cavity comprised the shim stack sequence. The total width of the shim setup was 15 cm. The orifice width for first extrusion orifices leading to the first cavity was 0.408 mm and the orifice width for second extrusion orifices leading to the second cavity was 0.816 mm. The land spacing between the first and second orifices was 0.408 mm.

The inlet fittings on the two end blocks were each connected to a conventional 1.25 inch (3.17 cm) single-screw extruder. The extruder feeding the first cavity was loaded with polyolefin elastomer resin pellets (obtained under the trade designation ENGAGE 8200 from the Dow Chemical Company, Midland, MI). The extruder feeding the second cavity was also loaded with polyolefin elastomer resin pellets (ENGAGE 8200). The extruder temperatures were set to 325 °F (162.8 °C) at the inlet and 375 °F (190.5 °C) at the outlet. The die temperature was set at 385 °F (196.1 °C). The extruder feeding the first cavity had a speed of 17.1 rpm and the extruder feeding the second cavity had a speed of 9 rpm.

The net was extruded vertically into a takeaway nip. The nip consisted of a metal nip roll surface and a silicone tool roll surface, with the temperatures set to 25 °C and 50 °C respectively. The cavities in the tool roll surface were circular in cross section with a diameter of 0.5 mm at the surface of the roll, a diameter of 0.3 mm at a depth of 1.5 mm, and a diameter of 0 mm at a depth of 2.0 mm. The cavities were arranged with center to center spacing of 1.35 mm in the axial roll direction, and 0.675 mm spacing in the circumferential direction. The nip pressure was sufficient to fill the cavities to a height of 0.300 mm. Polymers were extruded from the two cavities at appropriate flow rates to make a net and the take away speed was 6 feet (1.83 m) per minute.

A scanning electron microscope image (SEM, 10kV x22) of the resulting net is shown in FIG. 7. The plurality of protrusions were positioned on alternating strands of the net and the interconnected strands were oriented in the x-direction. The resulting net had a base thickness (strand height HI of Fig. 1) range of about 0.28-0.33 mm; strand width (T of Fig.1) ranges of about 0.36-0.48 mm; openings with width ranges of about 0.21-0.43 mm and length ranges of about 1.37-2.56 mm; protrusions with width at base (W2 of Fig. 3) of about 0.25-0.56 mm and height (H2 of Fig. 3) ranges of about 0.25-0.30 mm; and feature spacing (E of Fig. 3) ranges of about 0.51-0.65 mm.

### Example 4

A 2.5 cm by 2.5 cm section of the net of Example 1 was prepared and the surface of the net opposite from the surface containing protrusions was modified by corona treatment for 15-20 seconds using a hand-held unit with rastering motion (Model BD-20 Laboratory Corona Treater, Electro-Technic Products Company, Chicago, IL). One surface of a 2.5 cm by 2.5 cm (12 mm thick) pad of GRANUFOAM polyurethane foam (V.A.C. Granufoam Dressing Medium, KCI Incorporated, San Antonio, TX) was also modified using the corona treatment procedure described above.

A 2.5 cm by 2.5 cm section of 3M #2477 Double-Coated TPE Silicone/Acrylic adhesive tape (3M Company, Maplewood, MN) that had been perforated (1 mm diameter perforations patterned 3 mm center-to-center) was prepared. The paper release liner was removed and the exposed adhesive surface was heated for 10-20 seconds with hot air from an electric heat gun. The adhesive tape was edge aligned and applied to the corona treated surface of the foam pad. Next, the plastic release liner was removed from the tape and the corona treated surface of the net was edge aligned and applied to the exposed adhesive surface. Hand pressure was applied to the resulting laminate for a few seconds followed by placement of a 0.46 Kg weight on the laminate overnight. The weight was removed to provide the finished laminated article.

### Example 5

A double sided acrylic adhesive transfer tape (3M 300LSE tape #9472LE, 3M Company) was perforated through all layers in a repeating hexagonal pattern with 5 mm diameter perforations spaced 1 cm center-to-center. A 2.5 cm by 2.5 cm section of the net of Example 1 was prepared and the surface of the net opposite from the surface containing protrusions was modified by corona treatment for 15-20 seconds using a hand-held unit with rastering motion (Model BD-20 Laboratory Corona Treater). One surface of a 2.5 cm by 2.5 cm (12 mm thick) pad of GRANUFOAM polyurethane foam (V.A.C. Granufoam Dressing Medium) was also modified using the corona treatment procedure described above.

One of the release liners was removed from a 2.5 cm by 2.5 cm section the double sided adhesive transfer tape and the exposed adhesive surface was heated for 10-20 seconds with hot air from an electric heat gun. The adhesive was edge aligned and applied to the corona treated surface of the foam pad. Next, the second release liner was removed and the corona treated surface of the net was edge aligned and applied to the exposed adhesive surface. Hand pressure was applied to the resulting laminate for a few seconds followed by placement of a 0.46 Kg weight on the laminate overnight. The weight was removed to provide the finished laminated article.

## Claims

1. An article, comprising:
a network of interconnected polymeric strands; wherein each of the interconnected polymeric strands has a first surface adapted to contact a tissue site; wherein at least one of the interconnected polymeric strand has a plurality of protrusions extending from the first surface of the interconnected polymeric strands; wherein only one of any two adjacent polymeric strands has a plurality of protrusions extending from the first surface of the interconnected polymeric strands; and wherein at least one of the interconnected polymeric strands is non-linear; and
a plurality of openings between adjacent interconnected polymeric strands.

2. The article of claim 1, wherein at least one of the interconnected polymeric strands is linear.

3. The article of any one of claims 1 to 2, wherein the network comprises alternating non-linear polymeric strands and linear polymeric strands.

4. The article of any one of claims 1 to 3, wherein the non-linear polymeric strand has a sinusoidal curve.

5. The article of any one of claims 1 to 4, wherein the tensile strength of the article parallel to x direction is greater than the tensile strength of the article parallel to y direction.

6. The article of any one of claims 1 to 5, wherein the tensile strength of the article parallel to x direction is more than 2.23 Mpa.

7. The article of any one of claims 1 to 6, wherein the polymeric strands comprises an elastomeric polymer.

8. The article of claim 7, wherein the elastomeric polymer is selected from polyolefins or polyurethanes.

9. The article of any one of claims 1 to 8, wherein the protrusions have a height of 100 µm to 1000 µm.

10. The article of any one of claims 1 to 9, wherein the protrusions have a width of 10 µm to 1000 µm.

11. The article of any one of claims 1 to 10, wherein a ratio of the height to width of the protrusions is no more than 1:1

12. The article of any one of claims 1 to 11, wherein the density of protrusions extending from the first surface is less than 1,000/square inch.

13. The article of any one of claims 1 to 12, wherein aspect ratio of the openings is greater than 1:1.

14. The article of any one of claims 1 to 13, wherein essentially all the interconnected polymeric strands are non-linear.

15. A system, comprising:
the article of any one of claims 1 to 14; and
a reduced pressure source fluidly connected to the openings of the article to deliver the reduced pressure through the openings, between the protrusions, and to the tissue site.

## Patentansprüche

1. Ein Artikel, umfassend:
ein Netzwerk aus miteinander verbundenen Polymersträngen; wobei jeder der miteinander verbundenen Polymerstränge eine erste Oberfläche aufweist, die angepasst ist, um eine Gewebestelle zu kontaktieren; wobei mindestens einer der miteinander verbundenen Polymerstränge eine Mehrzahl von Vorsprüngen aufweist, die von der ersten Oberfläche der miteinander verbundenen Polymerstränge ausgehen; wobei nur einer von zwei beliebigen benachbarten Polymersträngen eine Mehrzahl von Vorsprüngen aufweist, die von der ersten Oberfläche der miteinander verbundenen Polymerstränge ausgehen; und wobei mindestens einer der miteinander verbundenen Polymerstränge nichtlinear ist; und
eine Mehrzahl von Öffnungen zwischen benachbarten miteinander verbundenen Polymersträngen.

2. Der Artikel nach Anspruch 1, wobei mindestens einer der miteinander verbundenen Polymerstränge linear ist.

3. Der Artikel nach einem der Ansprüche 1 bis 2, wobei das Netzwerk abwechselnde nichtlineare Polymerstränge und lineare Polymerstränge umfasst.

4. Der Artikel nach einem der Ansprüche 1 bis 3, wobei der nichtlineare Polymerstrang eine sinusförmige Kurve aufweist.

5. Der Artikel nach einem der Ansprüche 1 bis 4, wobei die Zugfestigkeit des Artikels parallel zur x-Richtung größer ist als die Zugfestigkeit des Artikels parallel zur y-Richtung.

6. Der Artikel nach einem der Ansprüche 1 bis 5, wobei die Zugfestigkeit des Artikels parallel zur x-Richtung mehr als 2,23 MPa beträgt.

7. Der Artikel nach einem der Ansprüche 1 bis 6, wobei die Polymerstränge ein elastomeres Polymer umfassen.

8. Der Artikel nach Anspruch 7, wobei das elastomere Polymer aus Polyolefinen oder Polyurethanen ausgewählt ist.

9. Der Artikel nach einem der Ansprüche 1 bis 8, wobei die Vorsprünge eine Höhe von 100 µm bis 1000 µm aufweisen.

10. Der Artikel nach einem der Ansprüche 1 bis 9, wobei die Vorsprünge eine Breite von 10 µm bis 1000 µm aufweisen.

11. Der Artikel nach einem der Ansprüche 1 bis 10, wobei ein Verhältnis der Höhe zur Breite der Vorsprünge nicht mehr als 1:1 beträgt.

12. Der Artikel nach einem der Ansprüche 1 bis 11, wobei die Dichte von Vorsprüngen, die sich von der ersten Oberfläche aus erstrecken, weniger als 1000/Quadratinch beträgt.

13. Der Artikel nach einem der Ansprüche 1 bis 12, wobei das Seitenverhältnis der Öffnungen mehr als 1:1 beträgt.

14. Der Artikel nach einem der Ansprüche 1 bis 13, wobei im Wesentlichen alle miteinander verbundenen Polymerstränge nichtlinear sind.

15. Ein System, umfassend:
den Artikel nach einem der Ansprüche 1 bis 14; und
eine Unterdruckquelle, die in Fluidverbindung mit den Öffnungen des Artikels steht, um den Unterdruck durch die Öffnungen, zwischen den Vorsprüngen und an die Gewebestelle abzugeben.

## Revendications

1. Article, comprenant :
un réseau de brins polymères interconnectés ; dans lequel chacun des brins polymères interconnectés a une première surface conçue pour venir en contact avec un site de tissu ; dans lequel au moins un parmi les brins polymères interconnectés a une pluralité de parties saillantes s'étendant à partir de la première surface des brins polymères interconnectés ; dans lequel un seul parmi deux brins polymères adjacents quelconques a une pluralité de parties saillantes s'étendant à partir de la première surface des brins polymères interconnectés ; et dans lequel au moins un parmi les brins polymères interconnectés est non linéaire ; et
une pluralité d'ouvertures entre des brins polymères interconnectés adjacents.

2. Article selon la revendication 1, dans lequel au moins un parmi les brins polymères interconnectés est linéaire.

3. Article selon l'une quelconque des revendications 1 à 2, dans lequel le réseau comprend des brins polymères non linéaires et des brins polymères linéaires en alternance.

4. Article selon l'une quelconque des revendications 1 à 3, dans lequel le brin polymère non linéaire a une courbe sinusoïdale.

5. Article selon l'une quelconque des revendications 1 à 4, dans lequel la résistance à la traction de l'article parallèle à la direction x est plus grande que la résistance à la traction de l'article parallèle à la direction y.

6. Article selon l'une quelconque des revendications 1 à 5, dans lequel la résistance à la traction de l'article parallèle à la direction x est supérieure à 2,23 MPa.

7. Article selon l'une quelconque des revendications 1 à 6, dans lequel les brins polymères comprennent un polymère élastomère.

8. Article selon la revendication 7, dans lequel le polymère élastomère est choisi parmi des polyoléfines ou polyuréthanes.

9. Article selon l'une quelconque des revendications 1 à 8, dans lequel les parties saillantes ont une hauteur de 100 µm à 1000 µm.

10. Article selon l'une quelconque des revendications 1 à 9, dans lequel les parties saillantes ont une largeur de 10 µm à 1000 µm.

11. Article selon l'une quelconque des revendications 1 à 10, dans lequel un rapport de la hauteur à la largeur des parties saillantes n'est pas supérieur à 1:1

12. Article selon l'une quelconque des revendications 1 à 11, dans lequel la densité de parties saillantes s'étendant à partir de la première surface est inférieure à 1000/pouce au carré.

13. Article selon l'une quelconque des revendications 1 à 12, dans lequel le rapport d'aspect des ouvertures est supérieur à 1:1.

14. Article selon l'une quelconque des revendications 1 à 13, dans lequel sensiblement tous les brins polymères interconnectés sont non linéaires.

15. Système, comprenant :
l'article de l'une quelconque des revendications 1 à 14 ; et
une source de pression réduite reliée fluidiquement aux ouvertures de l'article pour distribuer la pression réduite à travers les ouvertures, entre les parties saillantes, et au site de tissu.
